# EUROPÄISCHE PATENTANMELDUNG

(11) **EP 1 235 071 A1**
(43) Veröffentlichungstag der Anmeldung: **28.08.2002**
(21) Anmeldenummer: 01104218.1
(22) Anmeldetag: 22.02.2001
(51) Int. Cl.: G01N 33/36

(54) **Verfahren zum Prüfen der Reiss- und/oder Dehnungseigenschaften eines Garns**

(71) Anmelder: Gebrüder Loepfe AG, CH-8623 Wetzikon (CH)
(72) Erfinder: Scherer, Heinrich, 8344 Bäretswil (CH)
(74) Vertreter: Blum, Rudolf Emil Ernst

(57) **Zusammenfassung**

Zum Messen der Reiss- und/oder Dehnungseigenschaften eines Garns (2) wird beim Umspulen von den Copsen (1) auf die Kreuzspule (2) eine Messung durchgeführt. Hierzu wird auf das Garn (2) eine Zugspannung ausgeübt und es wird die Kraft gemessen, die z.B. zum Zerreissen notwendig ist. Dies erlaubt es, die Messung zu automatisieren. Eine manuelle Entnahme aus dem Fabrikationsprozess entfällt.

## Beschreibung

Die Erfindung betrifft ein Verfahren zum Prüfen der Reiss- und/oder Dehnungseigenschaften eines Garns und eine Umspulvorrichtung zur Durchführung dieses Verfahrens gemäss Oberbegriff der unabhängigen Ansprüche.

Reiss- und Dehnungseigenschaften sind wichtige Parameter eines Garns. Normalerweise werden Sie gemessen, indem Garnabschnitte z.B. in Form von Cops dem Fabrikationsprozess entnommen und im Labor geprüft werden. Dort wird das Garn in einem Messprozess unter Zugspannung gesetzt und gedehnt bzw. zerrissen, so dass ein Dehnungs-Kraft-Diagramm oder zumindest die zum Zerreissen notwendige Kraft aufgenommen werden kann. Derartige Prüfungen sind jedoch relativ aufwendig, so dass sie nur sporadisch durchgeführt werden können.

Es stellt sich deshalb die Aufgabe, ein Verfahren der eingangs genannten Art bereitzustellen, welches mit weniger Aufwand verbunden ist.

Diese Aufgabe wird vom Verfahren gemäss Anspruch 1 gelöst.

Erfindungsgemäss findet der Messprozess also während eines Fabrikationsschrittes des Garns statt, und zwar wird das Garn vor dem Aufspulen auf eine Zielspule in regelmässigen oder unregelmässigen Abständen dem Messprozess unterworfen. Dies erlaubt es, die Messung zu automatisieren. Eine manuelle Entnahme von Garn aus dem Fabrikationsprozess zur Messung in einem Labor entfällt.

Vorzugsweise umfasst der Messprozess eine Reissprüfung, d.h. das Garn wird zerrissen. Um es sodann weiter auf die Zielspule aufzuspulen, wird die Rissstelle repariert, was in der Regel durch Spleissen geschieht.

Die Messung findet vorzugsweise beim Umspulen des Garns von einer Quellspule auf die Zielspule statt. Beispielsweise kann dies beim Umspulen vom Cops auf die Kreuzspule geschehen. Entsprechende Umspulstationen sind in der Regel bereits mit Messköpfen für Garnparameter und Steuerungselektronik ausgestattet, so dass sich an diesem Ort auch eine Dehnungs- bzw. Reissprüfung gut integrieren lässt.

Besonders gut lässt sich der Messprozess mit einer Garnreinigung kombinieren. Bei einer Garnreinigung wird am Garn eine Qualitätsmessung durchgeführt, welche Garnfehler, wie zum Beispiel Dickstellen, Dünnstellen oder Verunreinigungen, erfasst. Wird ein Garnfehler detektiert, so wird die Fehlstelle herausgeschnitten und das Garn wird von einem Spleisser wieder verspleisst. Wenn am gleichen Ort die Reissfestigkeit des Garns gemessen wird, so kann der gleiche Spleisser verwendet werden, um das Garn auch nach der Reissprüfung zu reparieren. Vorzugsweise werden die Dehnungs- bzw. Reisseigenschaften nach dem Entfernen einer Fehlstelle gemessen.

Beim Entfernen einer Fehlstelle wird die Zielspule in der Regel angehalten. Am besten sollte der Messprozess stattfinden, bevor die Zielspule wieder Betriebsgeschwindigkeit erreicht, so dass der Zeitverlust gering bleibt.

Das Verfahren kann auch verwendet werden, um Spleissstellen zu prüfen, wie sie z.B. beim Herausschneiden von Garnfehlern oder beim Übergang zwischen zwei Spulen entstehen. Mit konventionellen Messverfahren können Reiss- und Dehnungseigenschaften von Spleissstellen in Routinemessungen praktisch nicht erfasst werden.

Das Verfahren wird vorzugsweise in einer Umspulvorrichtung durchgeführt, in welcher das Garn von den Copsen auf die Kreuzspule umgespult wird. Die Messandordnung wird dabei im Garnweg zwischen dem Cops und der Kreuzspule angeordnet.

Weitere bevorzugte Ausführungen, Vorteile und Anwendungen des Verfahrens ergeben aus den abhängigen Ansprüchen sowie der nun folgenden Beschreibung anhand der Figuren. Dabei zeigen:
Fig. 1 eine Umspulstation zur Durchführung des Verfahrens,
Fig. 2 eine erste Ausführung einer Zugvorrichtung und
Fig. 3 eine zweite Ausführung einer Zugvorrichtung.

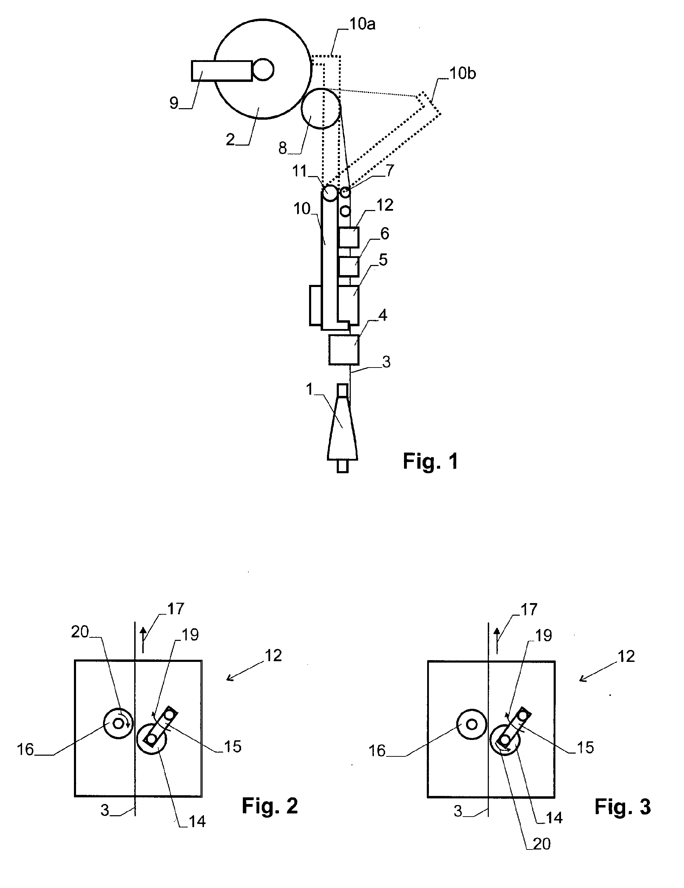

In einer bevorzugten Ausführung wird die vorliegende Erfindung bei einer Umspulstation durchgeführt, wie sie z.B. in Fig. 1 dargestellt ist. In einer derartigen Umspulvorrichtung bzw. Umspulstation wird nacheinander das Garn mehrerer Cops 1 (Quellspulen) auf eine Kreuzspule 2 (Zielspule) umgespult. Die Copse 1 kommen dabei in der Regel von den Spinnstellen, in welchen das Garn hergestellt wurde.

Zwischen Cops 1 und Kreuzspule 2 durchläuft das Garn 3 in konventionellen Umspulstationen zuerst eine Brems- und Vorreinigungseinrichtung 4, einen Spleisser 5, einen Messkopf 6 eines Garnreinigers und eine Paraffiniereinrichtung 7. Von dort gelangt das Garn zu einer Fadenführungstrommel 8. Die Fadenführungstrommel 8 besitzt Nuten zum Führen des Garns beim Aufwickeln auf die Kreuzspule 2. Sie ist angetrieben und in Reibkontakt mit der Kreuzspule 2, um diese in Drehung zu versetzen. Ferner ist an der Kreuzspule 2 eine Bremse 9 angeordnet, um die Kreuzspule 2 bei einem Unterbruch schnell abzubremsen. Ausserdem besitzt die Umspulstation ein um einen Drehpunkt 11 schwenkbares Saugorgan 10. Das Saugorgan 10 befindet sich im Normalbetrieb in der in Fig. 1 durchgezogen dargestellten Stellung. Nach einem Garnbruch oder dem Entfernen einer Fehlstelle kann es in eine obere, gestrichelt dargestellte Stellung 10a geschwenkt werden, wo es ein Garnende von der Kreuzspule 2 absaugt. Sobald es das Garnende aufgenommen hat, wird es über eine gestrichelt dargestellte Stellung 10b heruntergeklappt und zieht dabei das Garn wieder in die Komponenten der Umspulstation ein.

Im Normalbetrieb der Umspulstation überwacht der Messkopf 6 die Qualität des Garns. Wird ein Garnfehler detektiert, so wird das Garn im Bereich des Messkopfs 6 geschnitten. Sodann wird die Kreuzspule 2 gestoppt. Das Garnende wird von der Kreuzspule 2 abgesogen und zum Spleisser 5 geführt. Dort wird die fehlerhafte Stelle entfernt und die Garnenden werden wieder miteinander verspleisst. Danach beginnt das Umspulen erneut.

Zusätzlich zu den konventionellen Komponenten einer Umspulstation ist eine Zugvorrichtung 12 vorgesehen, welche im vorliegenden Fall nach dem Messkopf 6 angeordnet ist. Eine bevorzugte Ausführung der Zugvorrichtung 12 ist in Fig. 2 dargestellt. Die Zugvorrichtung 12 bildet eine Messanordnung zum Prüfen der Reiss- und/oder Dehnungseigenschaften des Garns.

Die Zugvorrichtung 12 besitzt ein frei drehendes erstes Laufrad 14, welches an einem elektromagnetisch kontrollierten Schwenkarm 15 angeordnet ist. Das erste Laufrad 14 kann durch Bewegen des Schwenkarms gegen ein zweites Laufrad 16 bewegt werden. Das zweite Laufrad 16 kann mit einem (nicht dargestellten) Motor in Drehung versetzt werden, und zwar so, dass es eine Kraft gegen die Laufrichtung 17 des Garns 3 ausübt.

Im normalen Umspulbetrieb ist das erste Laufrad 14 vom zweiten Laufrad 16 weggeschwenkt, so dass das Garn 3 keines der Laufräder 14, 16 berührt und nicht von der Zugvorrichtung 12 beeinflusst wird.

Die Zugvorrichtung 12 erlaubt es, in einem Messprozess die Dehnungs- und/oder Reisseigenschaften des Garns 3 zu bestimmen. Hierzu wird die Kreuzspule 2 vorzugsweise angehalten und mit der Bremse 9 festgehalten. Sodann wird das erste Laufrad 14 in Richtung des Pfeils 19 gegen das zweite Laufrad verschwenkt und das zweite Laufrad 16 wird in Richtung des Pfeils 20 angetrieben, so dass es eine Zugkraft auf das Garn 3 ausübt.

Die so erzeugte Zugkraft wird gemessen, indem der Strom durch den Antriebsmotor des zweiten Laufrads 16 bestimmt wird. Alternativ hierzu kann die Zugvorrichtung 12 auf einem Kraftsensor gelagert sein, was eine direkte Kraftmessung erlaubt.

Eine alternative Ausführung der Zugvorrichtung 12 ist in Fig. 3 dargestellt. Im Gegensatz zur Zugvorrichtung nach Fig. 2 ist hier das erste, schwenkbar angeordnete Rad 14 zur Drehung angetrieben, während das zweite Rad 16 keinen eigenen Antrieb besitzt. Indem das schwenkbar angeordnete Rad zur Drehung angetrieben ist und bei einer im wesentlichen tangential und gleichgerichtet zur Laufrichtung 17 des Garn gerichteten Schwenkbewegung (Pfeil 19) in Berührung mit dem zweiten Rad gebracht wird, erzeugt die Drehung des zweiten Rades gleichzeitig eine Andruckkraft auf das erste Rad 16.

Vorzugsweise wird die Schwenkbewegung (Pfeil 19) und die Drehbewegung (Pfeil 20) vom gleichen Antrieb erzeugt.

In einer einfachsten Ausführung können auf diese Weise die Reisseigenschaften des Garns bestimmt werden, d.h. die Kraft, die benötigt wird, um das Garn zu zerreissen. In einer aufwendigeren Ausführung kann auch ein Kraft-Weg-Diagramm vor dem Zerreissen des Garns aufgenommen werden, woraus die Dehnungseigenschaften bestimmt werden können.

Nach einer Reissprüfung durch die Zugvorrichtung 12 kann das Garn, wie nach dem Herausschneiden eines Garnfehlers, wieder zusammengefügt werden, indem die Enden im Spleisser 5 miteinander verbunden werden.

Messprozesse zum Bestimmen der Reiss- bzw. Dehnungseigenschaften des Garns können zu verschiedenen Zeitpunkten stattfinden.

Vorzugsweise wird eine Messung am Anfang und/oder am Ende eines Cops durchgeführt. Zu diesem Zeitpunkt muss die Kreuzspule sowieso gestoppt werden, so dass der Messprozess ohne wesentlichen Zeitverlust durchgeführt werden kann. Bei einer Messung nach dem Abspulen eines Cops wird das Garnende zuerst mit dem Saugorgan 10 von der Kreuzspule aufgenommen und in die Zugvorrichtung 12 eingeführt. Dann wird ein Messprozess durchgeführt. Danach wird das Ende des Garns wiederum mit dem Saugorgan 10 aufgenommen und sodann mit dem Garn des nächsten Cops verspleisst. Auf diese Weise entsteht keine zusätzliche Spleissstelle.

Zusätzlich oder alternativ hierzu kann z.B. ein Messprozess nach einem Reinigerschnitt durchgeführt werden, d.h. wenn das Garn 3 zum Entfernen einer Fehlstelle abgebremst wird. Auch in diesem Fall kann ein wesentlicher Zeitverlust vermieden werden.

Es ist auch möglich, in vorgegebenen Abständen einen Messprozess durchzuführen. Dies ermöglicht eine regelmässige Kontrolle, führt jedoch zu einem gewissen Zeitverlust, falls das Garn für die Messung abgebremst werden soll, und zu einer zusätzlichen Spleissverbindung.

Ein Messprozess kann auch manuell ausgelöst werden.

Mit dem vorliegenden Verfahren ist es, wie bereits erwähnt, auch möglich, Spleissstellen auszumessen, z.B. nach einem Copswechsel oder einem Reinigerschnitt. Hierzu wird das Garn nach dem Spleissen so weit bewegt, dass sich die Spleissstelle zwischen der Zugvorrichtung 12 und der Kreuzspule 2 befindet. Nun wird ein Messprozess der oben beschriebenen Art durchgeführt. Auf diese Weise kann eine Fehlfunktion des Spleissers 5 frühzeitig erkannt werden.

Wie erwähnt wird die Kreuzspule 2 vorzugsweise abgebremst, bevor ein Messprozess durchgeführt wird. Dies erlaubt es, eine genaue Messung durchzuführen. Die Kreuzspule 2 hält in diesem Falle das Garn fest, während die Zugvorrichtung 12 das Garn von der Kreuzspule 2 wegzieht.

Theoretisch ist es jedoch denkbar, die Messung ohne vorheriges Abbremsen der Kreuzspule auszuführen, indem das Garn durch die Zugvorrichtung 12 bei laufender Kreuzspule 2 festgehalten wird. Entsprechende Messungen werden jedoch weniger genau.

In einer weiteren Ausführung kann die Zugvorrichtung 12 als einfache Klemmvorrichtung ausgestaltet sein, die das Garn lediglich festhält, ohne aktiv daran zu ziehen. In diesem Fall kann die Zugkraft durch den Antrieb der Kreuzspule 2 ausgeübt werden. Es zeigt sich jedoch, dass eine so durchgeführte Messung weniger genau ist, da eine Bewegung zwischen der Fadenführungstrommel 8 und dem Garn während der Messung einen vorzeitigen Garnbruch bewirken kann.

In einer anderen Ausführung kann die Zugvorrichtung 12 am Saugorgan 10 angeordnet werden. In diesem Falle findet der Messprozess nach einem Garnunterbruch statt, z.B. bei einem Copswechsel oder einem Reinigerschnitt. Wenn das Saugorgan 10 das Garn 3 von der Kreuzspule 2 aufgenommen hat und sich z.B. in der Stellung 10b befindet, hält die Zugvorrichtung 12 das Garn fest und es wird, bei stillstehender Kreuzspule 2, durch Schwenken des Saugorgans 10 ein Zug ausgeübt und die Zugkraft wird gemessen.

In den soweit beschriebenen Beispielen wurde das Garn an einem Ende von der Kreuzspule 2 gehalten und am anderen Ende von der Zugvorrichtung 12. Zwischen diesen beiden Punkten wurde ein Zug ausgeübt. Für quantitative Dehnungsmessungen hat diese Anordnung den Nachteil, dass der Endpunkt der getesteten Garnlänge auf der Seite der Kreuzspule 2 nicht genau definiert ist. Für genauere Messungen kann deshalb auch eine zweite Zugvorrichtung vorgesehen werden, welche z.B. in der Nähe der Kreuzspule 2 angeordnet wird, und das Garn an einem definierten Punkt festhält. Sodann wird das Garn zwischen den beiden Zugvorrichtungen gedehnt.

In den obigen Beispielen findet der beschriebene Messprozess bei einer Umspulstation statt. Es ist jedoch auch möglich, die Messung an anderen Orten durchzuführen. Beispielsweise kann bei Openend-Spinnverfahren, bei denen das frisch gesponnene Garn direkt, d.h. ohne Umweg über einen Cops, auf die Kreuzspule aufgespult wird, der Messprozess vor der Kreuzspule stattfinden. Bei modernen Openend-Spinnmaschinen sind mehrere Spinnstellen nebeneinander angeordnet. Es ist ein verfahrbarer Anspinnwagen vorgesehen, der eine Anspinnvorrichtung trägt und bedarfsweise bei einer Spinnstelle positioniert werden kann. In diesem Falle kann die oben beschriebene Zugvorrichtung z.B. ebenfalls auf diesem Wagen angeordnet werden, so dass sie zusammen mit diesem zwischen mehreren Spinnstellen verfahrbar ist. Ebenso ist es denkbar, für mehrere Umspulstationen eine einzige, verfahrbare Zugvorrichtung 12 vorzusehen, die bedarfsweise zwischen den Stationen verfahren wird.

## Patentansprüche

1. Verfahren zum Prüfen der Reiss- und/oder Dehnungseigenschaften eines Garns (3) in Messprozessen, wobei in jedem Messprozess das Garn (3) unter Zugspannung gesetzt und gedehnt und/oder zerrissen wird, **dadurch gekennzeichnet, dass** in einem Fabrikationsschritt das Garn (3) auf eine Zielspule (2) aufgespult wird, wobei vor dem Aufspulen auf die Zielspule (2) das Garn (3) in Abständen dem Messprozess unterworfen wird.

2. Verfahren nach Anspruch 1, **dadurch gekennzeichnet, dass** das Garn (3) im Messprozess zerrissen wird und sodann wieder zusammengefügt, insbesondere gespleisst, wird, um es weiter auf die Zielspule (2) aufzuspulen.

3. Verfahren nach einem der vorangehenden Ansprüche, **dadurch gekennzeichnet, dass** der Messprozess beim Umspulen des Garns (3) von einer Quellspule (1) auf die Zielspule (2) durchgeführt werden, insbesondere beim Umspulen von einem Cops auf eine Kreuzspule.

4. Verfahren nach Anspruch 3, **dadurch gekennzeichnet, dass** am Anfang und/oder am Ende der Quellspule (1) einer der Messprozesse durchgeführt wird, und insbesondere dass mehrere Quellspulen (1) nacheinander auf eine Zielspule umgespult werden.

5. Verfahren nach einem der vorangehenden Ansprüche, **dadurch gekennzeichnet, dass** die Zielspule (2) vor jedem Messprozess abgebremst, insbesondere angehalten, wird.

6. Verfahren nach einem der vorangehenden Ansprüche, **dadurch gekennzeichnet, dass** am Garn (3) vor der Zielspule (2) eine Qualitätsmessung durchgeführt wird und Fehlstellen aus dem Garn herausgeschnitten und mit einem Spleisser (5) verspleisst werden, und dass der Spleisser (5) auch verwendet wird, um das Garn (3) nach dem Messprozess zu verspleissen.

7. Verfahren nach Anspruch 6, **dadurch gekennzeichnet, dass** nach dem Herausschneiden einer Fehlstelle einer der Messprozesse stattfindet.

8. Verfahren nach Anspruch 7, **dadurch gekennzeichnet, dass** die Zielspule zum Herausschneiden der Fehlstelle angehalten wird und ein Messprozess stattfindet, bevor die Zielspule (2) wieder Betriebsgeschwindigkeit erreicht.

9. Verfahren nach einem der vorangehenden Ansprüche, **dadurch gekennzeichnet, dass** im Garn (3) bei einem Spulenwechsel oder bei Auftreten einer Fehlstelle eine Spleissstelle erzeugt wird und dass die Spleissstelle einem der Messprozesse unterworfen wird zur Prüfung der Festigkeit der Spleissstelle.

10. Verfahren nach einem der vorangehenden Ansprüche, **dadurch gekennzeichnet, dass** im Messprozess die Zielspule angetrieben und das Garn (3) vor der Zielspule festgehalten wird.

11. Verfahren nach einem der vorangehenden Ansprüche, **dadurch gekennzeichnet, dass** im Messprozess die Zielspule (2) das Garn (3) festhält und eine Zugvorrichtung das Garn von der Zielspule wegzieht.

12. Verfahren nach einem der vorangehenden Ansprüche, **dadurch gekennzeichnet, dass** nach einem Garnunterbruch ein Ende des Garns (3) mit einem beweglichen Saugorgan (10) von der Zielspule (2) aufgenommen wird, wobei das Saugorgan (10) im Messprozess verwendet wird, um einen Zug auf das Garn (3) auszuüben, und insbesondere dass beim Ausüben des Zugs das Garn (3) mit einer Zugvorrichtung (12) am Saugorgan festgehalten wird.

13. Verfahren nach einem der vorangehenden Ansprüche, **dadurch gekennzeichnet, dass** eine Zugvorrichtung (12) zum Erzeugen der Zugspannung verfahrbar zwischen mehreren Umspul- oder Spinnstellen angeordnet wird und zum Durchführen der Messprozesse zwischen den Umspulbzw. Spinnstellen verfahren wird.

14. Verfahren nach einem der vorangehenden Ansprüche, **dadurch gekennzeichnet, dass** die Zugspannung durch eine Zugvorrichtung (12) erzeugt wird, die ein erstes und ein zweites Rad (14, 16) aufweist, wobei zum Erzeugen der Zugspannung das erste Rad (14) durch eine Schwenkbewegung (19) im wesentlichen gleichgerichtet zur Laufrichtung (17) des Garns zum zweiten Rad (16) hin bewegt, das Garn (3) zwischen den Rädern beaufschlagt und das zweite Rad zur Drehung angetrieben wird.

15. Umspulvorrichtung zum Umspulen eines Garns von mindestens einem Cops auf eine Kreuzspule **gekennzeichnet durch** eine zwischen dem Cops (1) und der Kreuzspule (2) angeordnete Messanrodnung (12) zum Prüfen der Reiss- und/oder Dehnungseigenschaften des Garns.
